Europäisches Patentamt

European Patent Office

Office européen des brevets

(11) Publication number: **0 407 586 A1**

(12) **EUROPEAN PATENT APPLICATION**
published in accordance with Art.
158(3) EPC

(21) Application number: 89910188.5

(22) Date of filing: 13.09.89

(86) International application number:
PCT/JP89/00936

(87) International publication number:
WO 90/02761 (22.03.90 90/07)

(51) Int. Cl.5: **C07K 15/04**, C12N 5/00,
C12N 15/00, C12P 21/00,
G01N 33/574, G01N 33/577,
A61K 39/395, //(C12P21/00,
C12R1:91)

(30) Priority: 14.09.88 JP 228340/88

(43) Date of publication of application:
16.01.91 Bulletin 91/03

(84) Designated Contracting States:
AT BE CH DE FR GB IT LI NL SE

(71) Applicant: **TEIJIN LIMITED**
6-7, Minamihonmachi 1-chome Chuo-ku
Osaka-shi Osaka 541(JP)

(72) Inventor: **ENDO, Noriaki**
**15-14, Tokura 1-chome Kokubunji-shi**
**Tokyo 185(JP)**
Inventor: **MASUHO, Yasuhiko**
**20-2, Tamadaira 5-chome Hino-shi**
**Tokyo 191(JP)**
Inventor: **HARA, Takeshi**
**35-10, Nakayama 3-chome Hachiooji-shi**
**Tokyo 192-03(JP)**

(74) Representative: **Votier, Sidney David et al**
**CARPMAELS & RANSFORD 43, Bloomsbury**
**Squareare**
**London WC1A 2RA(GB)**

(54) HUMAN MONOCLONAL ANTIBODY AND PROCESS FOR ITS PREPARATION.

(57) The invention relates to human monoclonal antibody of IgM type which reacts with oligosaccharides, glycoprotein or glycolipid having a terminal sugar chain structure of galactose($\beta1 \rightarrow$ 3)N-acetylgalactosamine and/or galactose ($\beta1 \rightarrow$ 3)N-acetylglucosamine and which reacts with human cancer cells, and hybridoma 11-50 yielding the antibody. This monoclonal antibody can be used for diagnosis of cancer, etc.

- 1 -

S P E C I F I C A T I O N

Title of the invention:
Human monoclonal antibody and the preparation method
-thereof

Field of the art:
The present invention relates to a human monoclonal antibody (abbreviated to MCA hereinafter) selectively reacting with human cancers and a preparation method thereof. One of the object according to the present invention is to provide a MCA which is reactive with human cancers as well as glycoprotein antigens or glycolipid antigens associated with human cancers and useful in the diagnosis and treatment for the cancers.

Background of the art:
The diagnosis and treatment for cancers have not yet been established and a variety of attempts have been made for development of more effective methods. Particularly, the development of diagnosis and treatment with antibodies have been pursued by many scientists, since the preparation of highly pure MCA has become possible by means of the hybridoma technique established by Milstein and Koehler.

One of the most important things in such diagnosis anu treatment is the specificity of antibodies. In other words, the antibodies useful for such diagnosis and treatment are desired to be reactive with cancer cells and/or cancer tissues, but unreactive or scarcely reactive with normal cells and/or normal tissues or reactive only with a relatively restricted range of normal cells and/or normal tissues. Additionally, in many cases it is desirable that such antibodies are reactive with a variety of cancer cells and/or cancer tissues in view of versatility.

- 2 -

A certain kind of antibodies against saccharide moiety of glycolipids such as gangliosides or glycoprotein have a possibility of satisfying such requirements [for example, see Hakomori and Kannagi: J. Natl. Cancer Inst., 71, 231-251 (1983)].

For example, Thomsen-Friedenreich antigen (abbreviated to TF-antigen hereinafter) is a glycoprotein or glycolipid antigen which contains characteristically galactose ($\beta 1 \rightarrow 3$) N-acetylgalactosamine (abbreviated to Gal$\beta 1 \rightarrow 3$GalNAc hereinafter) at the chain terminal as the antigenic determinant and it is a tumor-associated antigen which distributes in a variety of human cancers in an out-exposed state [G. F. Springer, Science, 224: 1198-1206 (1984)]. Therefore, the MCA which is reactive with glycoprotein or glycolipid bearing Gal$\beta 1 \rightarrow 3$ GalNAc is thought to be useful in the diagnosis and treatment for cancers.

Rahman and Longenecker [J. Immunol. 129, 2021-2024 (1982)] immunized mice with human erythrocytes having a glycoprotein whose saccharide terminal was modified to Gal$\beta 1 \rightarrow 3$GalNAc by sialidase treatment, then fused the mouse splenic cells with mouse myeloma cells to prepare a hybridoma producing a Gal$\beta 1 \rightarrow 3\alpha$GalNAc-specific MCA. But, later this hybridoma was reported to be unreactive with human cancer cells [cf. Longenecker et al., Int. J. Cancer, 33, 123-129 (1984)].

After that, however, they described the acquisition of the MCAs which were reactive with Gal$\beta 1 \rightarrow 3$GalNAc, and further reactive with human cancer cells [for example, J. Natl. Cancer Inst. 78, 489-496 (1987)]. Such MCAs can be expected to be useful in the diagnosis and treatment for cancers. But, it is highly probable that these MCAs could induce undesired side-effects, because they are mouse-derived MCAs which can be recognized as a foreign material, when given to human bodies. Thus, the creation of human-, but not mouse-

- 3 -

derived MCA which reacts with glycoprotein or glycolipid bearing Galβ1→3GalNAc as a terminal saccharide are desired.

On the contrary, it has been known that anti-TF antibodies exist in the serum of normal persons as a natural antibody mainly composed of IgM [Springer et al., Naturwissenschaften, 69, 346-348, (1982)], and such anti-TF antibodies could be prepared from human serum, for example, by means of an immune adsorption column using a saccharide relating to TF antigen. However, the content of the anti-TF antibodies in the serum is low and the sufficient supply should be very difficult, as long as the source is human blood. Therefore, the preparation of human-derived MCA which is reactive with the glycoprotein or glycolipid containing Galβ1 3GalNAc as well as with human cancer cells has been desired, but so far it has been unsuccessful.

Kabat et al. reported [(J. Immunol., 128, 540-544 (1982)] that, in a patient with bronchogenic lung cancer with an incidental gammopathy, the progressive lung cancer was regressed presumably by the monoclonal IgM antibody specifically reactive with lacto-N-tetraose [galactose (β1→3)N-acetylglucosamine(β1→3)galactose(β1→4)glucose (abbreviated to Galβ1→3GlcNAcβ1→3Galβ1→4Glc hereinafter)] which was concurrently produced in the body and that no recurrence of the cancer was observed for a long period of time. Thus, there is a possibility that an antibody reactive with oligosaccharides, or glycoproteins or glycolipids having the lacto-N-tetraose or galactose (β1→3) N-acetylglucosamine, (abbreviated to Galβ1→3GlcNAc, hereinafter), a partial structure of the lacto-N-tetraose, as a terminal saccharide chain is useful in the treatment for cancer. The preparation of human MCA with such reactivity by means of the hybridoma technique, for example, has been desired, but it is still unsuccessful.

- 4 -

In general, human-derived MCA is produced from a hybridoma which is obtained by cell fusion between mouse myeloma cells or human myeloma cells or other lymphoid established cell lines and human lymphocytes. Or it is also produced from the lymphoblasts which have been prepared by transforming human lymphocytes with EB virus. From 1980 to the present, a variety of human MCA preparations have been tried, but all of the processes bear their own respective problems. For example, the MCA production of a hybridoma obtained by cell fusion between mouse myeloma cells and human lymphocytes is unstable, and the cell fusion between human myeloma cells and human lymphocytes is extremely low in its efficiency. Further, the MCA production by the lymphoblasts obtained by EB virus is very small and unstable. Moreover, EB virus has a capability of causing tumors and its safety is problematic. Thus, there are not only many difficulties in the technology to establish a line of MCA-producing cells, but also a variety of obstacles to obtain human MCA selectively reacting with human cancers. One of the biggest problems is how to select and obtain the hybridoma producing human MCA specifically reacting with human cancers. If they are selected on the basis of their reactivities with the cancer cells themselves, generally the efficiency to obtain human MCA selectively reactive with human cancers is very low.

Disclosure of the invention:

The inventor have made intense study, in order to obtain human MCA which reacts with saccharide chain structures in antigens relating to tumors and reacts with human cancer cells, and found that such a hybridoma as to produce human MCA which reacts with oligosaccharides, glycoproteins or glycolipids bearing Galβ1→3GalNAc and/or Galβ1→3Glc-NAc as a saccharide chain terminal structure and reacts with

- 5 -

human cancer cells can be obtained by fusing human lymphocytes cultured in vitro in the presence of mitogen with mouse myeloma cells, selecting the fused cells according to their reactivity with glycoprotein or glycolipid having Gal$\beta$1$\to$3GalNAc and/or Gal$\beta$1$\to$3GlcNAc, then examining their reactivity with human cancer cells, and simultaneously investigating their reactivities with a variety of glycoproteins, glycolipids, and oligosaccharides. Then, the hybridoma and/or cell lines derived from the hybridoma have been cultured and said human MCA has been obtained from the culture supernatant. Thus, the human MCA according to the present invention is an antibody which reacts with glycoproteins having Gal$\beta$1$\to$3GalNAc as a saccharide chain terminal structure such as asialoglycophorin 4 and asialofetuin or glycolipids having the above-stated chain terminal structure such as ganglioside GM1, ganglioside GD1b or the like, and oligosaccharides having Gal$\beta$1$\to$3GlcNAc as a saccharide chain terminal structure such as lacto-N-tetraose or glycoproteins having the above-stated terminal saccharide chain structure such as bovine serum albumin conjugated with Gal$\beta$1$\to$3GlcNAc or glycolipids having the above-stated saccharide chain terminal group such as lactotetraosylceramide, and further reacts with human cancer cells such as human colon cancer cells and human breast cancer cells.

Best embodiment for the invention:

Human lymphocytes distribute in spleen, lymph nodes, peripheral blood, bone marrow, tonsil, adenoid and so on. According to the present invention, lymphocytes from any source can be used, but the lymphocytes from spleen, lymph nodes or tonsil are particularly suitable.

The 8-azaguanine-resistant cell line is advantageously used as mouse myeloma cells, and the lines of BALB/c mouse P3x65Ag8, P3-NS1/1-Ag4-1, P3x63AgU1, SP2/0Ag14, P3x63Ag8.6.

- 6 -

5.3, MPC11-45.6.TG1.7 and SP-1 can be employed.

According to the present invention, human lymphocytes are preferably cultured in vitro in the presence of mitogen before the fusion between the human lymphocytes and the mouse myeloma cells.

Any mitogen can be employed as long as it accelerates the differentiation and proliferation of the lymphocytes, and for example, pokeweed mitogen (PWM), B-cell growth factor (BCGF), protein A, phytohemaggultinin (PHA) and concanavalin A can be used. Particularly, 2 to 200 µg/ml of PWM or 1/100 to 1/3 volume of BCGF are preferably employed.

Further, the lymphocytes may be cultured in the presence of glycoprotein or glycolipid having Galβ1→3GalNAc and/or Galβ1→3GlcNAc as a saccharide chain terminal structure, or cells having the above-stated glycoprotein or glycolipid on their cell-surface as an antigen source.

The condition for lymphocyte culture is not particularly restricted, but the lymphocyte concentration is suitably $1 \times 10^5$ to $1 \times 10^7$ cells/ml; the culture temperature, 35 to 40°C and the culture time, 4 to 10 days, preferably 6 to 8 days. The culture medium containing human, bovine or equine serum and/or materials containing such serum components can be employed.

The human antibody-producing cells (lymphocytes) th obtained and mouse myeloma cells are fused according to a known method. For example, the lymphocytes and the myeloma cells were mixed at a ratio of 10 : 1 to 1 : 100, preferably 1 : 1 to 1 : 10 and stirred together with a suitable cell fusion solution, for example, about 35 % polyethylene glycol (molecular weight: about 1,000 to 6,000) and about 7.5 % dimethyl sulfoxide-containing RPMI 1640 at room temperature to 37°C for 1 to several minutes, then the mixture is gradually diluted with 10 % fetal bovine serum-containing RPMI 1640, washed, then the cell concentration is adjusted to 1

- 7 -

to 5 x $10^5$ cells/ml using HAT (hypoxanthine-aminopterin-thymidine) selective culture medium. The cell suspension were plated onto a 96-well plate with 0.2 ml each well and they are cultured in the air containing 5 % $CO_2$ at 35 to 38°C for 3 to 4 weeks. In the HAT culture media, only the hybridomas survive, but the 8-azaguanine-resistant myeloma cells and the myeloma-myeloma fusion cells cannot survive (the unfused antibody-producing cells died in a few days).

After cultivation, the antibody titer is determined and only the hybridomas producing the appropriate antibody were selected and isolated (cloning). The antibody titer in the culture medium is determined by, for example, the radioimmunoassay (RIA), enzyme-linked immunosorbent assay (ELISA) or immunofluorescence technique. A glycoprotein or glycolipid having Galβ1→3GalNAc as a saccharide chain terminal structure, for example, asialoglycophorin, asialofetuin, ganglioside GM1 or GD1b is used as an antigen to select the hybridoma producing MCA reactive with such antigens. Then, the culture supernatants of the hybridomas selected by such methods are examined on the reactivities with a variety of glycoproteins, glycolipids and oligosaccharides, and the reactivities with human cancer cells and human normal cells whereby the hybridomas producing the human MCA which react with oligosaccharides, glycoprotein or glycolipids having Galβ1→3GalNAc and/or Galβ1→3GlcNAc as a saccharide chain terminal structure, and reacts with human cancer cells are obtained efficiently. The reactivity with human cancer cells can be investigated using, for example, the RIA method, ELISA method or immunofluorescence method.

The mouse-human hybridoma producing human anti-human cancer MCA according to the present invention can be stored under freezing. Thus, when the cell line of such hybridoma and/or a cell line derived from the hybridoma is cultured by a suitable method in a large scale, the human MCA, the

- 8 -

target material according to the present invention can be obtained from the supernatant of the culture mixture.

The human MCA which has been obtained as stated above, according to the present invention, has the following characteristics. In other words, the human MCA according to the present invention is reactive with glycoproteins having Gal β1→3GalNAc and/or Galβ1→3GlcNAc as a saccharide chain terminal structure, for example, asialoglycophorin A, asialofetuin or bovine serum albumin conjugated with Galβ1→3GlcNAc, but unreactive at all or very slightly reactive with the glycophorin A and fetuin having a saccharide chain structure of sialic acid-binding Galβ1→3GlcNAc or glycoprotein not containing any Galβ1→3GalNAc and Galβ1→3GlcNAc, for example, bovine thyroglobulin or human α1-acidic glycoprotein from which sialic acid has been removed by acid treatment. As for the reactivity with gangliosides, it reacts with GM1 and GD1b having Galβ1→3GalNAc at their terminals, but does not react at all with GT1b having the terminal galactose where sialic acid is bound and GM2, GM3 or the like not containing Galβ1→3GalNAc. In the reactivities with oligosaccharides, the saccharides having Galβ1→3GlcNAc at their terminals such as lacto-N-tetraose and Galβ1→3GalNAcα1-OMe show strong reactivity.

As for the reactivity with cell lines of human origin, the human MCA according to the present invention is reactive with cancer cells such as a variety of colon cancer cells and breast cancer cells, but unreactive with normal cells such as fibroblasts and lymphocytes. Accordingly the human MCA prepared according to the present invention is an antibody which is characterized by the reactivity with oligosaccharides, glycoproteins or glycolipids having Galβ1→3GalNAc and/or Galβ1→3GlcNAc as a saccharide chain terminal structure as well as with human cancer cells. The anti-cancer human MCA having such characteristics has never previously

- 9 -

been prepared, and has been obtained for the first time by the present invention.

The present invention will be illustrated by the following examples in more detail.

Example 1

(1) Culture of human lymphocytes

Human amygdaline lymphocytes were suspended in culture medium A (RPMI1640 + 10 % fetal calf serum + 2 mM glutamine + 1 mM sodium pyruvate + 0.02 mg/ml serine + 80 µg/ml gentamicin). The cell concentration was $12 \times 10^5$ cells/ml. This cell suspension was poured into 5 wells in a culture plate (24 wells) with 1ml each well. PWM was added to each well so that the final concentration became 20 µg/ml, then the plate was placed in an atmosphere containing 5 % $CO_2$ at 37°C to continue the culture for 6 days.

(2) Cell fusion with mouse myeloma cell P3x63Ag8U1 line (abbreviated to P3U1)

P3U1 was cultured in culture medium B (GIT medium + 80 µg/ml gentamicin) beforehand. The cell concentration was $6 \times 10^5$ cells/ml, when the cells were used. The cultured lymphocytes stated above (the cells in 5 wells were put together) and P3U1 were washed separately with serum-free RPMI1640 twice. These lymphocytes and $2 \times 10^7$ P3U1 cells were combined in a test tube, centrifuged at 1,500 rpm for 5 minutes and the supernatant was discarded. The cell pellet was thoroughly dispersed by tapping the tube. Zero point five ml of polyethylene glycol solution (RPMI1640 5.75 ml + polyethylene glycol 1,000 3.5 ml + dimethyl sulfoxide 0.75 ml) was added to the test tube to allow the cells to be suspended calmly for 1minute. Zero point five ml and 1 ml of RPMI medium were then at one-minute interval. Four ml of HAT culture medium (GIT medium + 80 µg/ml gentamicin + 95 µM hypoxanthine + 0.4 µM aminopterin + 1.6 µM thymidine) was added twice at two-minute interval after then. Finally, HAT medium was added to prepare 100 ml of cell suspension. The cell suspension was plated onto 5 culture plates (96 wells each plate) and cultured in 5 % $CO_2$-containing air at 37°C. Every week, a half of the culture medium was exchanged with fresh HT medium (whose composition corresponds to aminopterin-free HAT culture medium) to obtain the hybridomas.

(3) Measurement of antibody activity

It was determined by the ELISA. As an antigen, 10 µg/ml asialofetuin (SIGMA) solution in 0.1 M borate buffer (pH 8.6) whose 100 µl was immobilized to each well of the Falcon microtest III plate (96 wells) was used. The supernatant of the hybridoma culture mixture was added to the plate with 50 µl every well and allowed to stand at room temperature for 1 hour. The wells were washed with 1 % bovine serum albumin (abbreviated to BSA hereinafter)-containing Hanks' buffer salt solution (abbreviated to HBSS-B) 3 times, then 100 µl of 0.6 mg/ml p-nitrophenyl phosphate solution in 1 M diethanolamine + 1 mM $MgCl_2$ (pH 9.8) were added. After 30 to 60 minutes, the absorbance at 405 nm wavelength was measured to select the hybridomas with antibody activity.

(4) Hybridoma cloning

Cloning was performed by the limiting dilution-culture method. The cells were taken out from the wells whose antibody activities to antiasialofetuin were positive, the cell number was counted, and they were inoculated into the wells, with 2 cells/well or 10 cells /well, using the culture medium B. After 2 weeks, when the cells sufficiently proliferated, the antiasialofetuin antibody titer of the supernatant was determined according to ELISA to select the MCA-

- 11 -

producing hybridomas.

Thus, hybridoma 11-50 was established. This hybridoma is steadily continuing the production of IgM type MCA. This hydridoma was deposited to the Fermentation Research Institute, Agency of Industrial Science and Technology, Ministry of International Trade and Industry, on September 29, 1988 (FERM P-10303).

Example 2

(1) Reactivity of the MCA produced by hybridoma 11-50 with a variety of glycoproteins

ELISA was conducted using, as an antigen, 4 kinds of glycoproteins, namely asialoglycophorin, asialofetuin, bovine thyroglobulin (these three from SIGMA) and asialo $\alpha$1-acidic glycoprotein which was prepared by treating human $\alpha$1-acidic glycoprotein (SIGMA) with 0.1N HCl at 80°C for 1 hour. These antigen proteins were prepared into 100, 10, 1 and 0.1 $\mu$g/ml solutions in 0.1 M borate buffer solution (pH 8.6) and immobilized to the wells, with 100 $\mu$l in each well, in the Falcon microtest III 96 well plate. Then, the following procedures were done according to those described in Example 1 - (3). The results are given in Table 1.

Table 1

| Glycoproteins | Protein Concentration ($\mu$g/ml) | | | |
| | 100 | 10 | 1 | 0.1 |
| --- | --- | --- | --- | --- |
| Asialoglycophorin | *1)2.32 | >2.90 | 2.03 | 0.87 |
| Asialofetuin | 1.16 | 0.87 | 0.58 | 0.29 |
| Bovine thyroglobulin | <0.1 | <0.1 | <0.1 | <0.1 |
| Asialo $\alpha$1-acidic glycoprot. | 0.29 | 0.29 | <0.1 | <0.1 |
| Gal$\beta$1→3GalNAc-BSA*2) | 2.32 | >2.90 | 2.32 | 1.45 |
| Gal$\beta$1→3GlcNAc-BSA*3) | 2.32 | >2.90 | 2.03 | 1.74 |

- 12 -

Notes: 1) the values represent the absorbance at 405 nm wavelength.
2) BSA conjugated with Galβ1→3GalNAc. (PIERCE)
3) BSA conjugated with Galβ1→3GlcNAc. (PIERCE)

The MCA produced by hybridoma 11-50 strongly reacted with asialoglycophorin and asialofetuin which have Galβ1→3GalNAc at the terminal, but did not react at all or scarcely reacted with bovine thyroglobulin bearing a large amount of Galα1→4Gal and asialo α1-acidic glycoprotein having a large amount of Galβ1→4GlcNAc. Additionally, the MCA strongly reacted with the glycoproteins of BSA conjugated with Galβ1→3GalNAc or Galβ1→3GlcNAc.

(2) Reactions of the MCA produced by hybridoma 11-50 with a variety of glycolipids

Glycolipids listed in Table 2 were dissolved in a chloroform-methanol (1 : 2) mixed solvent to prepare the solutions of 0.625 mg/ml, 1.25 mg/ml, 2.5 mg/ml and 5 mg/ml. The solutions were poured into the wells in the Falcon microtest III plate, with 50 μl in each well, dried in the air and used as an antigen to measure the reactivity according to Example 1-(3). The results are shown in Table 2.

The MCA produced by hybridoma 11-50 strongly reacted with gangliosides bearing Galβ1→3GalNAc at the chain terminal, GM1 and GD1b.

- 13 -

Table 2

| Glycolipids | Amounts of glycolipids (µg/well) | | | |
|---|---|---|---|---|
| | 0.25 | 0.125 | 0.063 | 0.031 |
| GM1 | *1) 1.4 | 0.8 | 1.0 | 0.8 |
| GM2 | <0.1 | <0.1 | <0.1 | <0.1 |
| GM3 | <0.1 | <0.1 | <0.1 | <0.1 |
| GD1B | 1.2 | 1.0 | 1.0 | 1.8 |
| GD3 | <0.1 | <0.1 | <0.1 | <0.1 |
| GT1b | <0.1 | <0.1 | <0.1 | <0.1 |
| CMH | <0.1 | <0.1 | <0.1 | <0.1 |
| CDH | <0.1 | <0.1 | <0.1 | <0.1 |
| CTH | <0.1 | <0.1 | <0.1 | <0.1 |
| Globoside | <0.1 | <0.1 | <0.1 | <0.1 |

Note: *1. These values represent the absorbance at 405 nm wavelength.

Example 3

Reactions with various kinds of human cells

(1) Reaction with cultured human cell lines

The MCA produced by hybridoma 11-50 was allowed to react with various kinds of cultured human cell lines. Further, anti-human IgM antibody labeled with fluorescein-isothiocyanate (abbreviated to FITC) was allowed to react to observe under a fluorescence microscope. Thus, said MCA was confirmed to be strongly reactive with colon cancer cells such as LS174T or HT 29, lung cancer cells such as KNS-62 and breast cancer cells such as T47D.

(2) Reaction with human lymphocytes treated with sialidase

- 14 -

The reactivity of said MCA produced by hybridoma 11-50 with human spleen cells treated with 1U/ml of sialidase (SIGMA, type V) at 37°C for 45 minutes as well as untreated human spleen cells was examined by the indirect immunofluorescence technique described in Example 3-(1). Consequently, said MCA was proved to be strongly reactive with only the spleen cells treated with sialidase.


Example 4

(1) The inhibitory activities of glycoproteins on the reaction of the MCA produced by hybridoma 11-50 with human colon cancer cell line SW480

Fifty microliters of 30-fold diluted culture supernatant of hybridoma 11-50 with HBSS-B and fifty microliters of a variety of protein solutions diluted stepwise with HBSS-B (described in Table 3) were mixed beforehand, then 50 µl of the mixture was added to a 96-well plate to which human colon cancer cell line SW480 had been immobilized with glutaraldehyde, then allowed to stand at room temperature for 1 hour. The following procedures were done according to the method described in Example 1-(3). The results are shown in Table 3.

BSA conjugated with Galβ1→3GlcNAc revealed the strongest inhibitory activity, followed by asialoglycophorin, BSA conjugated with Galβ1→3GalNAc and asialofetuin in order.

On the contrary, no inhibitory activities was observed in bovine thyroglobulin, asialo α1-acidic glycoprotein and the glycophorin and fetuin having the sialic acid-bonded galactose residue at their terminals, at all.

- 15 -

## Table 3

| Glycoproteins | Concentrations (µg/ml) | | |
|---|---|---|---|
| (Inhibitors) | 500 | 250 | 125 |
| Galβ1→3GlcNAc-BSA | *1) 96 | 93 | 92 |
| Asialoglycophorin | not tested | 79 | 64 |
| Galβ1→3GalNAc-BSA | 78 | 61 | 59 |
| Asialofetuin | 46 | 36 | 40 |
| Bovine thyroglobulin | -1 | 4 | 16 |
| Asialo α1-acidic glycoprotein | 18 | 11 | 8 |
| Glycophorin | not tested | 13 | 11 |
| Fetuin | 15 | 13 | 6 |

Note: *1 The values represent the inhibitory activity in %, which was calculated from the following equation.

Inhibitory activity in % = [1 - ($A_{405}$ in the presence of an inhibitor)/($A_{405}$ in the absence of an inhibitor)]

(2) The inhibitory action of oligosaccharides on the reaction of the MCA produced by hybridoma 11-50 with human colon cancer cell line SW480

Various kinds of oligosaccharides (listed in Table 4) were diluted stepwise with HBSS-B and the solutions were used to conduct the evaluation according to the method described in Example 4-(1). The results are shown in Table 4.

Lacto-N-tetraose revealed the strongest inhibition, followed by Galβ1→3GalNAcα1-OMe. Other oligosaccharides did not show inhibitory effect in the concentration range where these 2 oligosaccharides exerted significant inhibitory activities.

- 16 -

### Table 4

| Oligosaccharides (inhibitors) | $IC_{50}$[1] (mM) |
| --- | --- |
| Lacto-N-tetraose[2] ($Gal\beta1{\rightarrow}3GlcNAc\beta1{\rightarrow}3Gal\beta1{\rightarrow}4Glc$) | 0.68 |
| $Gal\beta1{\rightarrow}3GalNAc\alpha1$-OMe[2] | 2.8 |
| $Gal\beta1{\rightarrow}4GlcNAc\beta1$-OMe[2] | >5.0 |
| Lactose ($Gal\beta1{\rightarrow}4Glc$) | 21 |
| Cellobiose ($Gal\beta1{\rightarrow}4Glc$) | >50 |

Notes: 1. the concentration of oligosaccharides inhibiting the bonding of MCA to the cancer cells by 50%.
2. available from BioCarb.

These results in (1) and (2) proved that the MCA produced by hybridoma 11-50 is reactive with the saccharide chain terminal structures of $Gal\beta1$ 3GlcNAc as well as $Gal\beta1$ 3GalNAc.

### Example 5

Identification of the isotype of the MCA produced by hybridoma 11-50

The identification of the isotype of human MCA was conducted by the ELISA using asialofetuin as an antigen and goat anti-human IgM or human IgG or human κ-chain or human λ-chain labeled with alkaline phosphatase as a secondary antibody. Thus, the isotype of said MCA was identified to be IgM, .

Possibility of industrial use

The human monoclonal antibody according to the present invention selectively reacts with human cancers, and it will be useful in the fields of diagnosis and treatment for cancers.

- 17 -

CLAIMS

1. Human monoclonal antibody which reacts with oligosaccharides, glycoproteins or glycolipids having galactose ($\beta1 \rightarrow 3$)N-acetylgalactosamine and/or galactose ($\beta1 \rightarrow 3$)N-acetylglucosamine as a saccharide chain terminal structure, and additionally reacts with human cancer cells.

2. Human monoclonal antibody according to claim 1 wherein it is the IgM type.

3. Human monoclonal antibody according to claim 2 wherein it is produced by hybridoma 11-50 which has been deposited to the Fermentation Research Institute, Agency of Industrial Science and Technology, Ministry of International Trade and Industry as FERM P-10303.

4. A preparation method for human monoclonal antibody which is characterized by fusing human lymphocytes with mouse myeloma cells, selecting, from the resulting hybridomas, the hybridoma producing human monoclonal antibody which reacts with oligosaccharides, glycoproteins or glycolipids having galactose ($\beta1 \rightarrow 3$) N-acetylgalactosamine and/or galactose ($\beta1 \rightarrow 3$)N-acetylglucosamine as a saccharide chain terminal structure, and additionally reacts with human cancer cells, then culturing such hybridoma and/or the cell line derived from the hybridoma, and collecting the human monoclonal antibody from the supernatant of the culture mixture.

5. A preparation method for human monoclonal antibody according to claim 4 wherein the hybridoma is hybridoma 11-50 which has been deposited to the Fermentation Research Institute, Agency of Industrial Science and Technology, Ministry of International Trade and Industry as FERM P-

- 18 -

10303.

6.    Hybridoma which produces human monoclonal antibody which reacts with oligosaccharides, glycoproteins or glyco-lipids having galactose ($\beta1\rightarrow3$) N-acetylgalactosamine and/or galactose ($\beta1\rightarrow3$)N-acetylglucosamine as a saccharide chain terminal structure, and additionally reacts with human cancer cells.

7.    Hybridoma according to claim 6 which has been deposited to the Fermentation Research institute, Agency of Industrial Science and Technology, Ministry of International Trade and Industry as FERM P-10303 (hybridoma 11-50).

# INTERNATIONAL SEARCH REPORT

International Application No  PCT/JP89/00936

## I. CLASSIFICATION OF SUBJECT MATTER (if several classification symbols apply, indicate all) ⁶

According to International Patent Classification (IPC) or to both National Classification and IPC

Int. Cl⁴   C07K15/04, C12N5/00, 15/00, C12P21/00, G01N33/574, 33/577, A61K39/395// (C12P21/00, C12R1:91)

## II. FIELDS SEARCHED

| Minimum Documentation Searched ⁷ | |
| --- | --- |
| Classification System | Classification Symbols |
| IPC | C07K15/04, C12N5/00, 15/00, C12P21/00, G01N33/574, 33/577, A61K39/395 |

Documentation Searched other than Minimum Documentation
to the Extent that such Documents are Included in the Fields Searched ⁸

## III. DOCUMENTS CONSIDERED TO BE RELEVANT ⁹

| Category * | Citation of Document, ¹¹ with indication, where appropriate, of the relevant passages ¹² | Relevant to Claim No. ¹³ |
| --- | --- | --- |
| P | JP, A, 63-258493 (Mitsui Toatsu Chemicals, Inc.), 25 October 1988 (25. 10. 88), (Family: none) | 1 - 7 |
| X | JP, A, 62-29599 (Mitsui Toatsu Chemicals, Inc.), 7 February 1987 (07. 02. 87), & EP, A1, 211368 | 1 - 7 |

* Special categories of cited documents: ¹⁰

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search | Date of Mailing of this International Search Report |
| --- | --- |
| December 7, 1989 (07. 12. 89) | December 25, 1989 (25. 12. 89) |
| International Searching Authority | Signature of Authorized Officer |
| Japanese Patent Office | |

Form PCT ISA '210 (second sheet) (January 1985)